# EUROPEAN PATENT APPLICATION

(11) **EP 2 561 803 A1**
(43) Date of publication of application: **27.02.2013**
(21) Application number: 12250060.6
(22) Date of filing: 16.03.2012
(51) Int. Cl.: A61B 5/00, A45D 44/00, A61B 5/107, A61B 5/11

(54) **Method of evaluating normalcy of lips and safety of lip treatments**

(30) Priority: 17.03.2011 US 201161453865 P
(71) Applicant: Medicis Pharmaceutical Corporation, Scottsdale, AZ 85256 (US)
(72) Inventor: Lin, Xiaoming, Scottdale, Arizona 85256 (US); Sanstead, Mary, Scottdale, Arizona 85256 (US); Ploeg, Heather Vander, Scottsdale, Arizona 85256 (US)
(74) Representative: Hargreaves, Timothy Edward

(57) **Abstract**

This disclosure relates to evaluating and objectively determining the relative normalcy of a human patient's lips, whether before or after a lip treatment, or independent from any lip treatment. The normalcy of a patient's lips is evaluated by examining a number of indicia and comparing the results to corresponding lip scales or reference descriptions. The normalcy may be evaluated before and after a lip treatment to determine the safety of the lip treatment.

## Description

### CROSS-REFERENCE TO RELATED APPLICATIONS

This Non-Provisional Patent Application claims priority to and the benefit of U.S. Provisional Patent Application Serial No. 61/453,865, entitled METHODS OF EVALUATING NORMALCY OF LIPS AND SAFETY OF LIP TREATMENTS and filed March 17, 2011, which is incorporated by reference herein in its entirety.

### FIELD

The disclosure relates generally to methods for evaluating normalcy of human patients' lips, and in exemplary embodiments, to evaluating the safety and effectiveness of lip treatments.

### BACKGROUND

The lip region, like the rest of the face, is subject to age-related tissue weakening and volume loss, which may be exacerbated by extrinsic factors such as sun exposure, repetitive facial movement and smoking. In addition, trauma to the lips may cause undesirable cosmetic damage. A wide range of treatments are available to repair damage (particularly age-related) to the lip area. These treatments include, for example, hyaluronic acid (HA) dermal fillers (e.g., Restylane^{®}), permanent fillers, autologous fat, and collagen-based fillers. Such treatments are often used for lip augmentation to reverse age-relined lip thinning or enhance lip thickness.

Lip repair and augmentation is typically performed in clinical setting. Evaluating the normalcy of the lips before and after such treatments, for example, to assess the overall health and wellness, functional and aesthetic improvement or the safety of such treatments, presents considerable difficulties for clinicians due to the lack of any standard objective protocols.

### SUMMARY

This disclosure relates to evaluating and objectively determining the relative normalcy of a human patient's lips, whether before or after a lip treatment, or independent from any lip treatment Among other things, the disclosure comprises a protocol for assessing the overall health and wellness of a human patient's lips which, in some embodiments, may be useful in evaluating the safety, aesthetic outcome, and effectiveness of a particular lip treatment for a human patient.

In an embodiment, this disclosure comprises visually evaluating at least one of a plurality of lip indicia of at least one of an upper lip and a lower lip, comparing lip indicia to a corresponding lip normalcy scale, wherein the corresponding lip normalcy scale comprises a series of classifications defining the relative normalcy of the corresponding lip indicia, and determining the overall normalcy of the at least one of an upper lip and a lower lip based on said steps of comparing lip indicia to lip scales.

In another embodiment, this disclosure comprises visually evaluating at least one of a plurality of lip indicia of at least one of an upper lip and a lower lip prior to performing a treatment, comparing the lip indicia to a corresponding lip scale prior to performing the treatment, wherein the corresponding lip scale comprises a series of classifications defining the relative normalcy of the lip indicia, performing a treatment, visually evaluating the lip indicia of at least one of an upper lip and a lower lip after performing the treatment,
comparing the lip indicia to a corresponding lip scale after performing the treatment, wherein the corresponding lip scale comprises a series of classifications defining the relative normalcy of the lip indicia, and determining the safety or aesthetic outcome of the treatment on the at least one of an upper lip and a lower lip based on said steps of comparing lip indicia to lip scales prior to and after the treatment.
In another aspect of the invention, there is provided a method for evaluating a human patient's lip comprising: selecting at least one lip indicium; visually evaluating each of the selected lip indicia of at least one of an upper lip and a lower lip; comparing each of the selected lip indicia to a lip scale, the lip scale comprising classifications defining the relative normalcy of each of the selected lip indicia; and determining the overall normalcy of the at least one of an upper lip and a lower lip based on the comparison of each of the selected lip indicia to the lip scale.
The method may further comprise performing a treatment on at least one of an upper and a lower lip of the human patient.
The step of performing a treatment may comprise injecting a filler before the step of selecting at least one lip indicium.
The method may further comprise generating data for each of the selected lip indicia.
The lip scale may comprise classifications of normal, mildly abnormal, moderately abnormal, and severely abnormal.
The selected lip indicia may include at least one of lip texture, lip firmness, lip symmetry, lip movement, lip function, and lip sensation
The step of visually evaluating each of the selected lip indicia may further comprise applying lateral compression and measuring the distortion of the surface of the at least one of an upper lip and a lower lip.
The step of visually evaluating each of the selected lip indicia may further comprise measuring the relative heights and widths of the left and right side of the vermilion of the at least one of an upper lip and a lower lip.
The step of visually evaluating each of the selected lip indicia may further comprise asking the human patient to speak a series of preselected words and determining the quantity of words spoken correctly, and comparing the quantity of words spoken correctly to a lip movement scale.
The step of visually evaluating each of the selected lip indicia may further comprise observing the patient suck a liquid through a plastic straw, and classifying the patient's ability to suck liquid through the plastic straw.
The step of visually evaluating each of the selected lip indicia may further comprise evaluating the device palpability of the at least one of an upper lip and a lower lip, and classifying the device palpability of the at least one of an upper lip and a lower lip.
The method may further comprise applying pressure to at least one point on the at least one of an upper lip and a lower lip using a monofilament device or a cotton wisp.
In another aspect of the invention there is provided a method for evaluating a lip treatment comprising: selecting at least one lip indicium; visually evaluating each of the selected lip indicia of at least one of an upper lip and a lower lip prior to performing a treatment; comparing each of the selected lip indicia to a lip scale prior to performing the treatment; wherein the lip scale comprises classifications defining the relative normalcy of each of the selected lip indicia; performing a treatment; visually evaluating each of the selected lip indicia of the at least one of an upper lip and a lower lip after performing the treatment; and comparing each of the selected lip indicia to the lip scale after performing the treatment.
The method may further comprise the step of determining the effectiveness of the treatment on the at least one of an upper lip and a lower lip based on said steps of comparing each of the selected lip indicia to the lip scale prior to and after the treatment.
The method may further comprise the step of determining the safety of the treatment on the at least one of an upper lip and a lower lip based on said steps of comparing each of the selected lip indicia to the lip scale prior to and after the treatment.
In another aspect of the invention there is provided a lip scale comprising: at least one normal grade and at least one abnormal grade, the at least one normal grade comprising at least one indicator demonstrating normal Hp attributes; and the at least one abnormal grade comprising at least one indicator demonstrating abnormal lip attributes.
The abnormal grade may further comprise grades of mild, moderate and severe.
The lip scale may comprise classifications defining the relative normalcy of at least one lip indicium selected from the group of firmness, texture, symmetry, movement, function, sensation, and mass formation.
In another aspect there is provided a computer program product storing a scale or at least part of a scale as claimed or described herein and/or comprising computer readable instructions that are executable to display a scale or at least part of a scale as claimed or described herein. The display of the scale or at least part of the scale may comprise displaying text or at least one image forming part of, or being representative of, the scale or part of the scale.
Any feature in one aspect may be applied as a feature in any other aspect, in any appropriate combination.

### BRIEF DESCRIPTION OF THE DRAWINGS

The subject matter of this disclosure is particularly pointed out and distinctly claimed in the concluding portion of the specification. A more complete understanding of this disclosure, however, may best be obtained by referring to the detailed description and claims when considered in connection with the drawing figures, wherein like numerals denote like elements and wherein:

Figure 1 is a flow sheet illustrating an exemplary embodiment;

Figure 2 is a flow sheet illustrating an exemplary embodiment; and

Figure 3 illustrates different points on the upper and lower lips to be evaluated by a clinician in a various embodiments.

### DETAILED DESCRIPTION

The detailed description of various embodiments herein makes reference to the accompanying drawing figures, which show various embodiments and implementations thereof by way of illustration and best mode, and not of limitation. While these embodiments are described in sufficient detail to enable those skilled in the art to practice the embodiments, it should be understood that other embodiments may be realized and that mechanical and other changes may be made without departing from the spirit and scope of this disclosure. Furthermore, any reference to singular includes plural embodiments, and any reference to more than one component may include a singular embodiment. Moreover, recitation of multiple embodiments having stated features is not intended to exclude other embodiments having additional features or other embodiments incorporating different combinations of the stated features. As used in this disclosure, the term "or" shall be understood to be defined as a logical disjunction (e.g., and/or) and shall not indicate an exclusive disjunction unless expressly indicated.

Given the above-deberibed issues facing those involved in lip repair and augmentation procedures, the inventors have determined that straightforward, clinically meaningful scales that can be used to measure the normalcy of the lips before and after lip treatments, including augmentation, would be highly beneficial. In this respect, the inventors set out to develop a protocol that clinicians and other medical professionals can use to evaluate the normalcy of the lips before and after such treatments. Such a protocol could also be used as the primary safety or effectiveness measurement for lip augmentation treatments or other lip treatments in clinical studies. This disclosure addresses this need.

As used herein, the term "effectiveness" is intended to include the efficacy of drug treatments as well as the effectiveness of device treatments (e.g., dermal fillers). The inventive protocol described herein is expected to provide numerous benefits to practitioners. The normalcy of the lip plays an important part in evaluation of the lip augmentation success and patient's satisfaction of the treatment. Evaluating the normalcy of a patient's lip function and appearance before and after treatment is using a number of different indicia is useful in determining the safety or effectiveness of the treatments. In addition, the use of a number of standardized scales provides a robust assessment tool, and improves the clinician's ability to communicate the effectiveness and aesthetic outcome of a treatment to the patient.

Notwithstanding the foregoing, persons skilled in the art will appreciate that the various methods described herein may be equally useful independent from any lip treatment.

Exemplary methods may be used in conjunction with methods to evaluate the effectiveness of lip treatments, such as those described in Serial No. 12/797,710, filed June 10, 2010, entitled "Methods for Measuring Change in Lip Size After Augmentation" and commonly assigned with this disclosure, which is hereby incorporated by reference in its entirety for all purposes.

An exemplary method of evaluating the normalcy of a patient's lips comprises visually inspecting the lips with regard to at least one indicium of normalcy. Indicia of normalcy may include, for example: texture, firmness, symmetry, device palpability, movement, function, sensation, and mass formation. For each indicium, a different characteristic of the patient's lips is observed and evaluated. Then, the evaluation of the indicium is compared to values on a corresponding lip normalcy scale, and a determination is made regarding the normalcy of the lips in regard to the specific indicium.

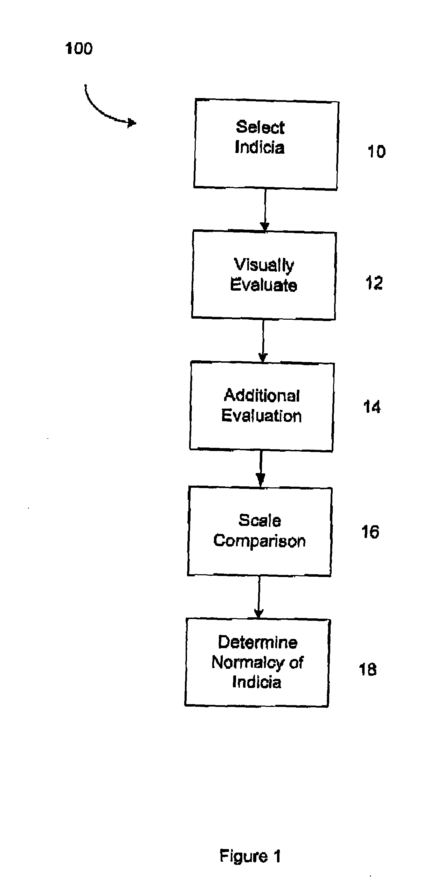

With initial reference to Figure 1, an exemplary embodiment is illustrated as lip normalcy evaluation method 100. The treating clinician or physician may employ normalcy evaluation method 100 by performing a select indicia step 10, by which to evaluate the normalcy of the patient's lips. Possible indicia include, among others, texture, firmness, symmetry, device palpability, movement, function, sensation, and mass formation. The clinician may select any number of indicia to evaluate. Such selection may depend on the type of treatment sought by the patient.

Once the clinician has selected the appropriate indicia, the clinician may then begin the visually evaluate step 12. For each indicium selected, the clinician may refer to a corresponding protocol which outlines the factors and considerations to evaluate for the indicium.

In various exemplary embodiments, the clinician can evaluate the patient's lip texture. The lip is visually inspected to determine the evenness of the lip's texture. Possible imperfections or irregularities may include, for example, visible undulations, coarseness, excessive smoothness, perpendicular lines, the presence of papules, or any other textural irregularities.

In various exemplary embodiments, the clinician can evaluate the patient's lip firmness. The lip is visually inspected to determine the lip's firmness. Possible imperfections or irregularities may include, for example, scars or lamps.

In various exemplary embodiments, the clinician can evaluate the patient's lip symmetry. The lip is visually inspected to determine the horizontal and vertical symmetry of the lip. Asymmetry may be visible, for example, as a height difference between the left and right side of the lips, or as a width difference between the two sides.

The clinician may also begin an additional evaluation step 14. Additional evaluation step 14 may comprise non-visual evaluation of lip indicia chosen in select indicia step 10. Non-visual evaluation methods may include, for example, physical manipulation of the lips, aural evaluation, and any other type of evaluation method that does not primarily rely upon a visual inspection.

In various exemplary embodiments, during additional evaluation step 14, the clinician may further evaluate device palpability within or stimulus response of the patient's lip. The lip is compressed laterally, and the surface distortion is observed arising from, for example, non-uniform device density or unexpected device lumpiness. Pressure may also be applied with an instrument such as, for example, a cotton-tipped applicator, toothpick, monofilament, or any other instrument capable of providing directed pressure to the lip. Pressure may be applied in more than one point on the lip to evaluate device palpability or stimulus response across the lip.

In various exemplary embodiments, the clinician may aurally evaluate the patient's lip movement. For example, the clinician may request that the patient pronounce a number of preselected words. The clinician may then evaluate the number or percentage of words pronounced correctly.

In various exemplary embodiments, the clinician can evaluate the patient's lip function. For example, the clinician may request that the patient suck a liquid, such as water, through a drinking straw.

In various exemplary embodiments, the clinician can evaluate the sensation capability of the patient's lip. For example, the clinician may provide stimulus to the lip and observe the lip's response to the stimulus. Such stimulus methods include, for example, providing sensation to the lip in at least one area by an instrument. For example, such instruments may include a monofilament wire or a cotton wisp.

In various exemplary embodiments, the clinician can evaluate mass formation in the patient's lip. For example, the clinician may visually or physically evaluate the patient's lip for lumps or aggregation of coherent material. If a lump or aggregation of coherent material exists in the lip, the clinician may record the mass. In an embodiment, such a mass can be documented as an adverse event or in the alternative, if the mass was found to have existed prior to treatment, such a mass can be charted in the patient's medical history.

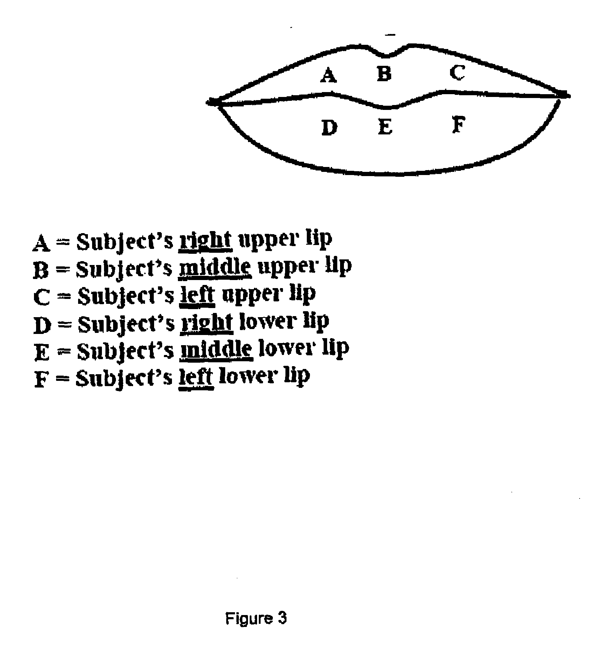

In connection with steps 14 and 16, the clinician can evaluate different points on the patient's lip, for example, 1, 2, 3, 4, 5, 6, or more distinct points on the patient's lip. By way of example, and with momentary reference to Figure 3, illustrated are three different points on each of the upper and lower lips to be evaluated by a clinician. In various embodiments, the clinician can evaluate distinct points on the patient's lip sequentially or in a random order.

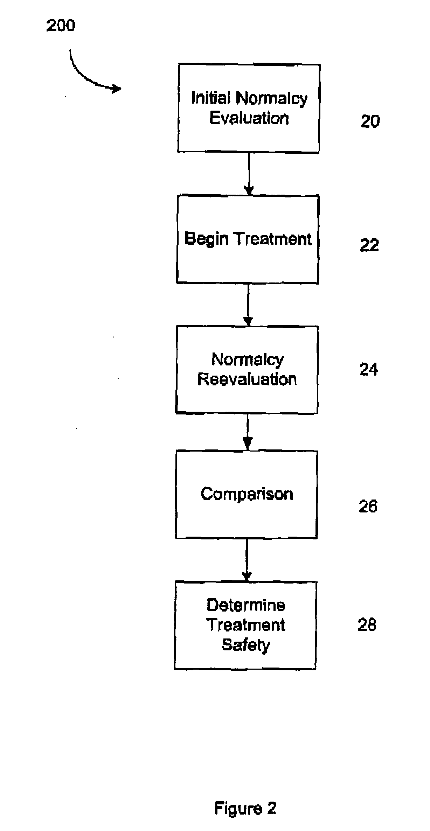

Turning back to Figure 2, in various exemplary embodiments, the clinician may perform a scale comparison step 16. Scale comparison step 16 comprises comparing the information about each evaluated indicia to the corresponding lip scale for that indicia. For example, the clinician can evaluate the lip firmness of the patient, In scale comparison step 16, the clinicians may compare the evaluation of the firmness of the patients lip to the lip firmness scale.

In various exemplary embodiments, the clinician may perform a determine normalcy of indicia step 18. For example, the clinician may classify the particular indicia of the patient's lip by matching the evaluation to a classification on the corresponding lip scale. For example, the clinician may compare the measured firmness of the patient's lip with the various classifications on the lip firmness scale. The clinician may then classify the patient's lip firmness according to the matching value or classification on the lip firmness scale. In such instances, the value or classification of each of the evaluated indicia may be recorded as data.

With reference to Figure 2, an exemplary method of determining the safety of a lip treatment 200 is illustrated.

In various exemplary embodiments, a clinician may begin determining the safety of a lip treatment method 200 by performing initial normalcy evaluation step 20. In an embodiment, initial normalcy evaluation step 20 comprises the same steps as lip normalcy evaluation method 100, as described herein. In an exemplary embodiment, the results of the initial normalcy evaluation step 20 may be used to determine what, if any, lip treatment or augmentation may be appropriate for the patient. For example, if the patient's lip fullness is determined to be abnormal or undesirable by patient during initial normalcy step 20, the patient may seek a treatment to improve lip fullness.

In various exemplary embodiments, if a treatment or augmentation is determined to be appropriate, and is elected by the patient, the clinician may begin treatment step 22. Exemplary treatments performed in treatment step 20 may include, for example, the use of hyaluronic acid (HA) dermal fillers (e.g., Restylane®), permanent fillers, autologous fat, or collagen-based fillers. However, any treatment, whether cosmetic or otherwise, which affects the appearance or function of the lips is in accordance with this disclosure.

In various exemplary embodiments, once a suitable time has passed subsequent to treatment step 20, the clinician may perform normalcy reevaluation step 24. Depending on the treatment step 20, suitable time may vary from hours to weeks. For example, if treatment step 20 comprises using HA dermal fillers, normalcy reevaluation step 24 may occur between 6 - 48 hours. In an embodiment, normalcy reevaluation step 24 comprises the same steps as initial normalcy evaluation step 20.

In various exemplary embodiments, after normalcy reevaluation step 24 has been completed, the clinician may perform comparison step 26. In an embodiment, comparison step 26 comprises comparing the results of initial normalcy evaluation step 20 with the results of normalcy reevaluation step 24. Comparison step 26 may be performed for each indicium chosen in select indicia step 10. For example, during comparison step 26, the clinicians may compare the results of initial normalcy evaluation step 20 and normalcy reevaluation step 26 for the patient's lip firmness.

In various exemplary embodiments, after comparison step 26 has been completed, the clinician may perform determine treatment safety step 28. In an embodiment, determine treatment safety step 28 comprises evaluating the results of comparison step 26. Determine treatment safety step 28 may be performed for each indicium chosen in select indicia step 10. For example, determine treatment safety step 28 may comprise evaluating the results of comparison step 26 in regards to the indicium of lip firmness. The clinician can evaluate the results of comparison step 26 with an initial goal described by the patient or clinician prior to treatment. For example, a patient may desire a significant improvement in lip firmness. The clinician may use the results of the determine treatment safety step 28 to express to the patient whether or not the improvement in lip firmness was attained by treatment step 20.

In accordance with exemplary embodiments, lip scales are provided. An exemplary lip scale may comprise a plurality of grades corresponding to a spectrum of lip normalcy, for example, encompassing normal and abnormal lips. According to embodiments, the lip scale comprises at least four grades, namely normal, mildly abnormal, moderately abnormal, and severely abnormal. Each grade may be accompanied by one or more drawings or photos of illustrative reference subject lips. In exemplary embodiments, different ethnicities, ages, or genders are represented by reference subject lips. Descriptive text useful to assist practitioners in assigning a grade to a patient's lips may supplement or substitute drawings or photos of illustrative reference subject lips.

In exemplary embodiments, photos / text are arranged horizontally, vertically, diagonally, or otherwise, for example in an overlapping or tiled layout. In exemplary embodiments, photos / text are grouped by grade of the scale. In exemplary embodiments, all photos / text are displayed together on a single tangible medium, for example, as a poster or chart, In other exemplary embodiments, only photos / text illustrative of a single grade are displayed together on a single medium, for example, as a flashcard. Persons skilled in the art will appreciate that, in addition to tangible mediums, photos / text may be displayed on a computer monitor, point of interaction device (personal digital assistant (such as an iPhone® or Blackberry®), cellular phone, kiosk, etc.), a projection device, or the like.

Returning to Figure 1, in an embodiment, scale comparison step 16 comprises comparing the results of visually evaluate step 12 and additional evaluation step 14 to standardized lip scales for each indicium selected by the clinician. Exemplary lip scales appear below in Tables 1-3.

Table 1 outlines an exemplary lip texture scale. The scale comprises grades of: normal, mildly abnormal, moderately abnormal, and severely abnormal. Each grade includes a description of lip texture condition that qualifies for the grade. For example, the texture of a lip will be classified as normal if the texture of the lip is even without visible undulations or excessive coarseness beyond that expected for the patient's stated age.

**Table 1 - Exemplary Lip Texture Scale**

| **NORMAL** | **ABNORMAL** | | |
|---|---|---|---|
| | **Mild** | **Moderate** | **Severe** |
| Texture of the lip was even without visible undulations or excessive coarseness beyond that expected for stated age. | The lip showed a single area of textural irregularity (a small papule, area of excess smoothness, focal absence of perpendicular lines) that could be visualized only with close inspection. | The lip showed more than one area of textural irregularity (a small papule, area of excess smoothness, focal absence of perpendicular lines) that could be visualized only with close inspection. | The lip showed two or more areas of textural irregularity (a small papule, area of excess smoothness, focal absence of perpendicular lines) that could be visualized at a conversational distance. |
| | | or | |
| | | The lip showed one area of textural irregularity (less than ¼ of the lip area) at conversational distance. | |
| | | | or |
| | | | The lip showed one area of textural insularity (more than ¼ of the lip area) at conversational distance. |

Table 2 outlines an exemplary lip firmness scale. The scale comprises grades of:
normal, mildly abnormal, moderately abnormal, and severely abnormal. Each grade includes a description of lip firmness condition that qualifies for that grade. For example, the firmness of a lip will be classified as normal if the lip is supple when compressed laterally and surface distorts readily with minimal pressure; pressure with a narrow diameter instrument (cotton-tipped applicator, toothpick etc) causes a focal depression in the surface of the lip; and upon palpation, lip is absent of abnormal structures such as scars or lumps.

**Table 2 - Exemplary Lip Firmness Scale**

| **NORMAL** | **ABNORMAL** | | |
|---|---|---|---|
| | **Mid** | **Moderate** | **Severe** |
| Lip was supple when compressed laterally and surface distorted readily with minimal pressure, Pressure with a narrow diameter instrument (cotton-tipped applicator, toothpick etc) caused a focal depression in the surface of the lip. Upon palpation, lip was absent of abnormal structures such as scars or lumps; normal product feel without being visible. | Up was slightly firm with lateral compression or required slightly greater than normal pressure to distort the surface. Upon palpation, an abnormal structure such as a scar or lump was felt, but was not visible. | Lip was firm with lateral compression or required distinctly greater than normal pressure to distort the surface or pressure with a narrow diameter instrument (cotton-tipped applicator or toothpick) caused a broader depression in the surface of the lip. Upon palpation, an abnormal structure such as a scar or lump was felt and was visible. | Lip was very firm with lateral compression or requires significantly greater than normal pressure to distort the surface. Upon palpation, an abnormal structure such as a scar or lump was felt and was visually distracting. |

Table 3 outlines an exemplary lip symmetry scale. The scale comprises grades of:
normal, mildly abnormal, moderately abnormal, and severely abnormal. Each grade includes a description of lip symmetry condition that qualifies for that grade, For example, the symmetry of a lip will be classified as normal if one side of the lip balances or mirrors the other side.

**Table 3 - Lip Symmetry Scale**

| **NORMAL** | **ABNORMAL** | | |
|---|---|---|---|
| | **Mild** | **Moderate** | **Severe** |
| One side of the lip balanced or mirrored the other side. | One side of the lip showed a 1 mm or less difference in height or a 1 mm or less difference in the length of the vermilion at repose. | One side of the lip showed a 1.1 mm to 2 mm difference in height or a 1.1 to 2 mm difference in the length of the vermilion at repose. | One side of the lip showed a greater than 2 mm difference in height or a greater than 2 mm difference in the length of the vermilion at repose. |

In exemplary embodiments only the upper lip is evaluated, while in other embodiments only the lower lip is evaluated. In yet other embodiments both lips are evaluated. In accordance with one aspect of an exemplary embodiment wherein both lips are evaluated, the respective scores may be averaged or only the least or most severe score may be recorded.

Other indicia, such as lip movement, lip function, and lip sensation, may be evaluated as well. In various exemplary embodiments, during additional examination step 14, a patient's lip movement is evaluated by assessing the patient's ability to properly pronounce a series of preselected words. In one embodiment, ten words are preselected. For each word properly pronounced, the patient's lip movement score is increased by one. The patient's lip movement may then be assessed in a range from zero to ten. In an embodiment, a score of eight or higher would constitute "normal" lip movement.

In various exemplary embodiments, during visual examination step 12 or additional examination step 14, a patients lip function is evaluated by assessing the patient's ability to suck liquid through a drinking straw. In an embodiment, a patient's lip function is classified as "abnormal" if they are unable to suck liquid through a drinking straw.

In various exemplary embodiments, during additional examination step 14, a patient's lip sensation is evaluated by applying pressure with an instrument to at least one point on the patient's lip and measuring the response, In an embodiment, the patient is blind-folded during the evaluation of lip sensation, and as pressure is applied, the patient is asked to indicate whether or not they feel the sensation. A patient's lip sensation may be classified as "abnormal" if they are unable to feel the pressure applied by the instrument.

In an exemplary embodiment, a monofilament, such as a Semmes-Weinstein 0.4G monofilament, is applied to at least one point on the patient's lip. In another exemplary embodiment, a cotton wisp is applied to at least one point on the patient's lip. However, any device or instrument which applies a non-damaging level of sensation to a patient's lip is in accordance with this disclosure.

An exemplary protocol may be used as the primary safety measurement for lip augmentation treatments in clinical studies, for example, in connection with seeking treatment approval from an administrative body. For example, an exemplary protocol may be used to evaluate the effects of a new type of filler by evaluating the pre- and posttreatment indicia such as lip firmness, texture, symmetry, device palpability, movement, function, sensation, and mass formation. Exemplary protocols may be used to evaluate the safety of any treatment which changes or affects a human patient's lip.

An exemplary protocol may be used as the primary effectiveness measurement for lip treatments. For example, an exemplary protocol may be used to determine the effectiveness of a lip augmentation treatment. The results of the protocol may be used to communicate the effectiveness of the treatment, as it relates to a number of indicia of normalcy, to the patient, The protocol may also help patients identify aspects of the treatment that were not as effective as anticipated. Used in this manner, the protocol may improve communication between the clinician and patient, and will likely contribute to patients' overall satisfaction with the lip treatment.

An exemplary protocol was actually used in a clinical study to evaluate the safety of an HA dermal filler. This was a randomized, evaluator blinded study of 180 subjects who were seeking lip augmentation from 12 investigational centers. At entry of the study, subjects were randomized in a 3:1 ratio to the HA dermal filler treatment or (2) no treatment.

Safety assessments included post treatment study adverse experiences at each visit and assessment of subject diary complaints. Lip texture, firmness and symmetry were scored at screening, 72 hours, and at Visit T, Weeks 2, 4, 8, 12, 16, 20, 24, 72 hours post treatment, Visit T 2, and 2 weeks and 4 weeks after 6 month treatment. These parameters were rated as "Normal" or "Abnormal." All abnormal ratings were further assessed as mild, moderate, or severe.

Subjects were assessed for lip movement, function, and sensation at screening, 72 hours, Visit T, and at Weeks 2, 4, 8, 12, 16,20,24, 72 hours post treatment, Visit T 2, and 2 weeks and 4 weeks after 6 month treatment. Lip movement was tested by assessing the ability of the subject to pronounce a preselected series of words. Lip function was tested by assessing the subject's ability to suck liquid through a straw. Lip sensation was tested using two methods: 1) monofilament test - assessing the subject's ability to feel the sensation of a 0.4G monofilament on 3 points on the upper lip and 3 points on the lower lip, and 2) cotton wisp test - assessing the subject's ability to feel the sensation of a cotton wisp on 3 points on the upper lip and 3 points on the lower lip. The 3 different points on the upper and lower lips were tested randomly. Subjects were blindfolded and asked to acknowledge sensation or lack of sensation at each point.

Device palpability was assessed at each scheduled post treatment visit and was assessed whether or not the palpability is the normal expected feel. Mass formation was assessed at all visits.

Table 4 summarizes the lip texture, firmness, symmetry, movement, function, sensation, and mass formation assessments at week 24 of the clinical study-

| **Lip Safety Assessment** | **Number of Assessments** | **Abnormal** | **Normal** |
|---|---|---|---|
| Lip Texture | 232 (Upper and Lower Lips Combined) | 0 | 232 |
| Lip Firmness | 232 (Upper and Lower Lips Combined) | 2 | 230 |
| Lip Symmetry | 232 (Upper and Lower Lips Combined) | 12 | 220 |
| Lip Movement - Did Subject Effectively Pronounce The Word? | 116 | 1 | 115 |
| Lip Function - Can Subject Drink/Suck Through A Straw Effectively? | 115 | 0 | 115 |
| Lip Sensation - Did Subject Feel the Monofilament? | 116 | 0 | 116 |
| Lip Sensation - Did Subject Feet the Cotton Wisp? | 116 | 0 | 116 |
| Mass Formation - Did Subject Have mass Formation? | 115 | 0 | 115 |

Table 5 summarizes the device palpability assessments at week 24 of the clinical study-

| Number of Assessments | Palpable Expected Feel | Palpable Unexpected Feel | Not palpable |
|---|---|---|---|
| 232 (Upper and Lower Lips Combined) | 143 | 0 | 89 |

To summarize, in the clinical study, exemplary lip safety assessments, such as lip texture, firmness, symmetry, movement, function, sensation, mass formation, and device palpability were evaluated at the screening visit, as appropriate, and at follow up visits. None of the lip assessments were abnormal or presented any safety concerns, providing evidence that the HA dermal filler was well tolerated for soft tissue augmentation in the lips.

Based in part upon the objectivity and results of the lip assessments described herein, the United States Food and Drug Administration ("USFDA") approved the HA dermal filler for lip augmentation. The USFDA also approved the lip assessments to measure safety outcomes in connection with dermal fillers for lip augmentation.

While the various embodiments set forth herein have been described with reference to dermal fillers, persons skilled in the art will appreciate that the methods may be applied equally to other lip treatments, such as a lip augmentation, or any other lip modification treatment (e.g., implants, surgical procedures, etc.) without departing from the spirit and scope of this disclosure. Moreover, benefits, other advantages, and solutions to problems have been described herein with regard to specific embodiments. However, the benefits, advantages, solutions to problems, and any elements that may cause any benefit, advantage, or solution to occur or become more pronounced are not to be construed as critical, required, or essential features or elements of the disclosure.

The scope of the disclosure is accordingly to be limited by nothing other than the claims, in which reference to an element in the singular is not intended to mean "one and only one" unless explicitly so stated, but rather "one or more." Moreover, where a phrase similar to 'at least one of A, B, or C' or 'at least one of A, B, and C' are used in the claims or specification, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. All structural, chemical, and functional equivalents to the elements of the above-described exemplary embodiments that are known to those of ordinary skill in the art are expressly incorporated herein by reference and are intended to be encompassed by the claims. Further, a list of elements does not include only those elements but may include other elements not expressly listed or inherent to such process, method, article, or apparatus.
Further embodiments are as set out in the following numbered clauses:-
Clause 1. A method for evaluating a human patient's lips comprising:
   selecting at least one lip indicium;
   visually evaluating each of the selected lip indicia of at least one of an upper lip and a lower lip;
   comparing each of the selected lip indicia to a lip scale, the lip scale comprising classifications defining the relative normalcy of each of the selected lip indicia; and
   determining the overall normalcy of the at least one of an upper lip and a lower lip based on the comparison of each of the selected lip indicia to the lip scale.
Clause 2. The method of clause 1, further comprising performing a treatment on at least one of an upper and a lower lip of the human patient.
Clause 3. The method of clause 2, wherein the step of performing a treatment comprises injecting a filler before the step of selecting at least one lip indicium.
Clause 4. The method of clause 1, further comprising generating data for each of the selected lip indicia.
Clause 5. The method of clause 1, wherein the lip scale comprises classifications of normal, mildly abnormal, moderately abnormal, and severely abnormal.
Clause 6. The method of clause 1, wherein the selected lip indicia includes at least one of lip texture, lip firmness, lip symmetry, lip movement, lip function, and lip sensation
Clause 7. The method of clause 1, wherein the step of visually evaluating each of the selected lip indicia further comprises applying lateral compression and measuring the distortion of the surface of the at least one of an upper lip and a lower lip.
Clause 8. The method of clause 1, wherein the step of visually evaluating each of the selected lip indicia further comprises measuring the relative heights and widths of the left and right side of the vermilion of the at least one of an upper lip and a lower lip.
Clause 9. The method of clause 1, wherein the step of visually evaluating each of the selected lip indicia further comprises asking the human patient to speak a series of preselected words and determining the quantity of words spoken correctly, and comparing the quantity of words spoken correctly to a lip movement scale.
Clause 10. The method of clause 1, wherein the step of visually evaluating each of the selected lip indicia further comprises observing the patient suck a liquid through a plastic straw, and classifying the patient's ability to suck liquid through the plastic straw.
Clause 11. The method of clause 1, wherein the step of visually evaluating each of the selected lip indicia further comprises evaluating the device palpability of the at least one of an upper lip and a lower lip, and
   classifying the device palpability of the at least one of an upper lip and a lower lip.
Clause 12. The method of clause 11, further comprising applying pressure to at least one point on the at least one of an upper lip and a lower lip using a monofilament device or a cotton wisp.
Clause 13. A method for evaluating a lip treatment comprising:
   selecting at least one lip indicium;
   visually evaluating each of the selected lip indicia of at least one of an upper lip and a lower lip prior to performing a treatment;
   comparing each of the selected lip indicia to a lip scale prior to performing the treatment;
   wherein the lip scale comprises classifications defining the relative normalcy of each of the selected lip indicia;
   performing a treatment;
   visually evaluating each of the selected lip indicia of the at least one of an upper lip and a lower lip after performing the treatment; and
   comparing each of the selected lip indicia to the lip scale after performing the treatment.
Clause 14. The method of clause 13, further comprising the step of determining the effectiveness of the treatment on the at least one of an upper lip and a lower lip based on said steps of comparing each of the selected lip indicia to the lip scale prior to and after the treatment.
Clause 15. The method of clause 13, further comprising the step of determining the safety of the treatment on the at least one of an upper lip and a lower lip based on said steps of comparing each of the selected lip indicia to the lip scale prior to and after the treatment.
Clause 16. A lip scale comprising:
   at least one normal grade and at least one abnormal grade,
   the at least one normal grade comprising at least one indicator demonstrating normal lip attributes; and
   the at least one abnormal grade comprising at least one indicator demonstrating abnormal lip attributes.
Clause 17. The lip scale of clause 16, wherein the abnormal grade further comprises grades of mild, moderate and severe.
Clause 18. The lip scale of clause 16, wherein the lip scale comprises classifications defining the relative normalcy of at least one lip indicium selected from the group of firmness, texture, symmetry, movement, function, sensation, and mass formation.

## Claims

1. A method for evaluating a human patient's lips comprising:
selecting at least one lip indicium;
visually evaluating each of the selected lip indicia of at least one of an upper lip and a lower lip;
comparing each of the selected lip indicia to a lip scale, the lip scale comprising classifications defining the relative normalcy of each of the selected lip indicia; and
determining the overall normalcy of the at least one of an upper lip and a lower lip based on the comparison of each of the selected lip indicia to the lip scale.

2. The method of claim 1, further comprising performing a treatment on at least one of an upper and a lower lip of the human patient, wherein
optionally:-
the step of performing a treatment comprises injecting a filler before the step of selecting at least one lip indicium.

3. The method of claim 1, further comprising generating data for each of the selected lip indicia.

4. The method of claim 1, wherein the lip scale comprises classifications of normal, mildly abnormal, moderately abnormal, and severely abnormal.

5. The method of claim 1, wherein the selected lip indicia includes at least one of lip texture, lip firmness, lip symmetry, lip movement, lip function, and lip sensation

6. The method of claim 1, wherein the step of visually evaluating each of the selected lip indicia further comprises applying lateral compression and measuring the distortion of the surface of the at least one of an upper lip and a lower lip.

7. The method of claim 1, wherein the step of visually evaluating each of the selected lip indicia further comprises measuring the relative heights and widths of the left and right side of the vermilion of the at least one of an upper lip and a lower lip.

8. The method of claim 1, wherein the step of visually evaluating each of the selected lip indicia further comprises asking the human patient to speak a series of preselected words and determining the quantity of words spoken correctly, and comparing the quantity of words spoken correctly to a lip movement scale.

9. The method of claim 1, wherein the step of visually evaluating each of the selected lip indicia further comprises observing the patient suck a liquid through a plastic straw, and classifying the patient's ability to suck liquid through the plastic straw.

10. The method of claim 1, wherein the step of visually evaluating each of the selected lip indicia further comprises evaluating the device palpability of the at least one of an upper lip and a lower lip, and classifying the device palpability of the at least one of an upper lip and a lower lip, and
optionally:-
the method further comprises applying pressure to at least one point on the at least one of an upper lip and a lower lip using a monofilament device or a cotton wisp.

11. A method for evaluating a lip treatment comprising:
selecting at least one lip indicium;
visually evaluating each of the selected lip indicia of at least one of an upper lip and a lower lip prior to performing a treatment;
comparing each of the selected lip indicia to a lip scale prior to performing the treatment;
wherein the lip scale comprises classifications defining the relative normalcy of each of the selected lip indicia;
performing a treatment;
visually evaluating each of the selected lip indicia of the at least one of an upper lip and a lower lip after performing the treatment; and
comparing each of the selected lip indicia to the lip scale after performing the treatment.

12. The method of claim 11, further comprising at least one of a) and b):-
a) the step of determining the effectiveness of the treatment on the at least one of an upper lip and a lower lip based on said steps of comparing each of the selected lip indicia to the lip scale prior to and after the treatment;
b) the step of determining the safety of the treatment on the at least one of an upper lip and a lower lip based on said steps of comparing each of the selected lip indicia to the lip scale prior to and after the treatment.

13. A lip scale comprising:
at least one normal grade and at least one abnormal grade,
the at least one normal grade comprising at least one indicator demonstrating normal lip attributes; and
the at least one abnormal grade comprising at least one indicator demonstrating abnormal lip attributes.

14. The lip scale of claim 13, wherein the abnormal grade further comprises grades of mild, moderate and severe.

15. The lip scale of claim 13, wherein the lip scale comprises classifications defining the relative normalcy of at least one lip indicium selected from the group of firmness, texture, symmetry, movement, function, sensation, and mass formation.
